Europäisches Patentamt

European Patent Office

Office européen des brevets

(19)

(11) Veröffentlichungsnummer: **0 212 529**
A1

(12) ## EUROPÄISCHE PATENTANMELDUNG

(21) Anmeldenummer: 86111094.8

(22) Anmeldetag: 11.08.86

(51) Int. Cl.⁴: **C 07 C 55/02**, C 07 C 51/50, C 07 C 55/14

(30) Priorität: 10.08.85 DE 3528805
26.11.85 DE 3541778

(43) Veröffentlichungstag der Anmeldung: 04.03.87
**Patentblatt 87/10**

(84) Benannte Vertragsstaaten: **FR GB**

(71) Anmelder: **Menk, Hermann, Dr. Dipl.-Chem.,
Gartenweg 17, D-8992 Wasserburg/Bodensee (DE)**

(72) Erfinder: **Menk, Hermann, Dr. Dipl.-Chem.,
Gartenweg 17, D-8992 Wasserburg/Bodensee (DE)**

(74) Vertreter: **Dipl.-Ing. Schwabe, Dr. Dr. Sandmair, Dr. Marx,
Stuntzstrasse 16 Postfach 86 02 45,
D-8000 München 86 (DE)**

(54) **Verfahren zur Verhinderung der Dunkelfärbung bei der Weiterverarbeitung der Nebenprodukte, die bei der Herstellung der Adipinsäure anfallen.**

(57) Es wird ein Verfahren zur Verhinderung der Dunkelfärbung bei der Weiterverarbeitung der Nebenprodukte, die bei
der Herstellung von Adipinsäure anfallen, beschrieben. Die
nachteilige Dunkelfärbung, die bei der Weiterverarbeitung der
Oxydationsprodukte des Cyclohexanols bei Erhitzen über ca.
150° auftritt, kann dadurch gelöst werden, indem man entweder der nach Abtrennung des Großteils der Adipinsäure verbleibenden Schmelze oder dem Gemisch der nach der Oxydation des Cyclohexanols erhaltenen Produkte Itaconsäure und/
oder Itaconsäureanhydrid in einer dem Stickstoff-Gehalt dieser Schmelze mindestens äquivalenten Menge zusetzt.

EP 0 212 529 A1

SCHWABE · SANDMAIR · MARX

PATENTANWÄLTE

STUNTZSTRASSE 16 · 8000 MÜNCHEN 80

Dr. Hermann Menk, Dipl.-Chem.

Gartenweg 17

8992 Wasserburg/Bodensee, BRD

Verfahren zur Verhinderung der Dunkelfärbung
bei der Weiterverarbeitung der Nebenprodukte,
die bei der Herstellung der Adipinsäure anfallen.

Die vorliegende Erfindung betrifft ein Verfahren zur Verhinderung der
Dunkelfärbung der Oxydations(-neben)-produkte des Cyclohexanols beim
Erhitzen über ca. 150°C durch Umsetzung eines gewissen Anteils der
entstandenen Oxydationsprodukte mit Itaconsäure und/oder Itaconsäureanhydrid.

- 2 -

V/Ma/mi

☎ (089) 98 82 72 -74
Telex: 5 24 560 Swan d

Telekopierer: (089) 98 30 49
Kalle Infotec 6350 Gr. II + III

Bankkonten: Bayer. Vereinsbank München 453 100 (BLZ 700 202 70)
Hypo-Bank München 4 410 122 850 (BLZ 700 200 11) Swift Code: HYPO DE MM
Postgiro München 653 43-808 (BLZ 700 100 80)

Zur Herstellung von z. B. Polyestern verwendet man als Ausgangsprodukt üblicherweise u. a. Adipinsäure. Die Herstellung der
Adipinsäure erfolgt dabei durch Salpetersäure-Oxydation von
Cyclohexanol, wobei man als Oxydationsprodukte überwiegend (ca.
95%) Adipinsäure und als Rest Glutar- und Bernsteinsäure mit
einem geringen Anteil ihrer Imide erhält. Nach den bisherigen
Verfahren konnte dieses Produktgemisch und damit ebenso gewisse daraus abgetrennte Anteile für die Herstellung von Polyestern für z. B. Polyurethanolelastomere und ungesättigten Polyesterharzen nicht unmittelbar weiterverwendet werden, da die
bereits erwähnten cyclischen Imide (2,5-Pyrrolidindion und
2,6- Piperidindion), zu einer unerwünschten starken Dunkelfärbung der Weiterverarbeitungsprodukte führen. Daher wurde
die Adipinsäure aus dem Gemisch der Oxydationsprodukte abgetrennt. Dabei erhält man mit ca. 95% Ausbeute reine Adipinsäure
und als Rest mit ca. 5% ein Gemisch aus etwa gleichen Teilen
Adipin-,Glutar- und Bernsteinsäure, das etwa 2% der Imide
der beiden letztgenannten Dicarbonsäuren enthält. Wie bereits
zuvor erwähnt, konnte bisher nur die reine Adinpinsäure zur
Herstellung der Polyester weiterverwendet werden, und das Dicarbonsäuregemisch mußte einer weniger wertvollen Verwendung
oder der Vernichtung zugeführt werden. Auch ein nachträglicher
Versuch der Hellfärbung  der End- bzw. Zwischenprodukte mit
Bleichmitteln wie z. B. Wasserstoffperoxid, Hypochlorite u. a.
führt nicht dazu, daß ein allgemein verwendbares Produkt er-

- 3 -

halten wird, wenn man zur Weiterverarbeitung das gesamte Gemisch der Oxydationsprodukte des Cyclohexanols oder das nach Abtennung eines großen Anteils der Adipinsäure erhaltene Dicarbonsäuregemisch einsetzt.

Die Aufgabe der vorliegenden Erfindung besteht nun darin, ein Verfahren anzugeben, durch das es möglich wird, die nachteilige Dunkelfärbung der bei der Oxydation des Cyclohexanols anfallenden Nebenprodukte zu beseitigen.

Eine weitere Aufgabe der vorliegenden Erfindung besteht darin, durch Verhinderung der Wirkung der bei der Oxydation des Cyclohexanols anfallenden Nebenprodukte das Verfahren so zu vereinfachen, daß man ggf. auf eine Abtrennung des Großteils der Adipinsäure aus dem Gemisch der Oxydationsprodukte verzichten kann und das gesamte Oxydationsgemisch unmittelbar nach einer einfachen, reinigenden Destillation weiterverarbeiten kann.

Überraschenderweise wurde nun gefunden, daß diese Aufgabe dadurch gelöst werden kann, indem man entweder der nach Abtrennung des Großteils der Adipinsäure verbleibenden Schmelze aus Glutar-, Bernstein- und Adipinsäure, oder dem Gemisch der nach der Oxydation des Cyclohexanols erhaltenen Produkte Itaconsäure und/oder Itaconsäureanhydrid zusetzt und damit den Nachteil der Dunkelfärbung der Endprodukte verhindert.

In einer bevorzugten Ausführungsform des Verfahrens setzt man der Schmelze, die nach Abtrennung des Großteils der Adipinsäure noch Bernstein-, Glutar- und einen Rest Adipinsäure enthält, eine dem Stickstoffgehalt dieser Schmelze mindestens äquivalente Menge Itaconsäure und/oder Itaconsäureanhydrid zu.

Nach Zusatz der/des Itaconsäure und/oder Itaconsäureanhydrids kann die Schmelze analog der Adipinsäure nach an sich bekannten Verfahren zu den erwünschten Endprodukten, wie z. B. den Polyurethanelastomeren und zu den ungesättigten Polyesterharzen

- 4 -

weiterverarbeitet werden.

Erfindungsgemäß wird auf 1 g-Atom Stickstoff der Schmelze mindestens 1 mol, vorzugsweise 1,15 mol Itaconsäure und/oder
Itaconsäureanhydrid zugegeben.

Weiterhin kann die Reaktion dadurch beschleunigt werden, daß
man der Schmelze 0,001 bis 5%, vorzugsweise 0,1 bis 2% aromatische Sulfonsäure, wie z. B. Benzol-, Toluol-, Naphthalin-,
Xylolsulfonsäure oder auch gleich wirkende azide Verbindungen
zusetzt, die in S.V. Vinogradova (Übersetzung von B.J. Hazzard,
Pergamon Press, 1965), S. 123 ff., 158 ff. und 174 ff. sowie in
der DDR-PS 62 449 aufgeführt sind .

Eine erfindungsgemäß erforderliche Obergrenze bezüglich des
Zusatzes der Itaconsäure und/oder Itaconsäureanhydrids besteht
nicht, jedoch wirkt sich ein Anteil von mehr als 15 % in der
Schmelze später nachteilig auf die Eigenschaften der Endprodukte aus, wie z. B. in gesättigten Polyesterharzen oder in da--
raus  gefertigten Polyurethanelastomeren, weil diese dann gegen
Ozonrißbildung empfindlich werden.

Ein weiterer Vorteil des erfindungsgemäßen Verfahrens liegt darin,
daß die cyclischen Imide in Fertigprodukten, z. B. in heiß-
oder kalthärtenden UP-Harzen oder in PUR-Elastomeren nicht als
Weichmacher oder mit anderen Extendereigenschaften fungieren
können, sondern an die Polymerkette chemisch gebunden vorliegen.

Zur Herstellung von Adipinsäure oxidiert man Cyclohexanol mit
konzentrierter Salpetersäure. Bei diesem Verfahren fallen neben
der Adipinsäure auch ihre beiden niederen Homologen, Glutar-
und Bernsteinsäure in einem beachtlichen Anteil an. In diesem
Verfahren wird die Salpetersäure zu einem kleineren Anteil soweit

- 5 -

reduziert, daß aus den zuletzt genannten Dicarbonsäuren cyclische Imide damit, nämlich 2,5-Pyrrolidindion (Bernsteinsäureimid) und 2,6-Piperidindion (Glutarsäureimid) gebildet werden. Danach wird die Adipinsäure mit hoher Ausbeute von den übrigen Bestandteilen getrennt. Dadurch erhält man einmal reine Adipinsäure (ca. 95%) und ein Dicarbonsäuregemisch, das etwa gleiche Anteile Adipin-, Glutar- und Bernsteinsäure, sowie zusätzlich noch etwa 2% der ebenerwähnten cyclischen Imide enthält.

Bei der Weiterverarbeitung dieses Nebenproduktgemisches bei höheren Temperaturen (ca. 150°C) zeigt sich nun, daß die Umsetzungsprodukte einer Dunkelfärbung unterliegen. Diese Dunkelfärbung tritt umso stärker auf, je höher die Temperatur und der Anteil der cyclischen Imide im Ausgangsproduktgemisch ist und je länger die Umsetzung dauert.

Erfindungsgemäß kann man die Dunkelfärbung dieses Gemisches dadurch verhindern, daß man der Schmelze des Ausgangsgemisches Itaconsäure und/oder Itaconsäureanhydrid (Methylenbernsteinsäure und/oder Methylenbernsteinsäureanhydrid) in der angegebenen Menge zusetzt.

Der Zusatz der Itaconsäure kann entweder bereits zum Rohprodukt der Cyclohexanoloxydation oder zum Dicarbonsäuregemisch in einer dem Stickstoffgehalt der jeweiligen Produktmischung mindestens äquivalenten Menge erfolgen, um dadurch die im Verlauf der weiteren Umsetzung eintretende Dunkelfärbung zu verhindern.

Erfindungsgemäß gibt man auf 1 g-Atom Stickstoff mindestens 1 mol, vorzugsweise 1,15 mol Itaconsäure und/oder Itaconsäureanhydrid zu.

Vorteilhafterweise begünstigt man die Reaktion, indem man der Gesamtmenge der Schmelze 0,1 % aromatische Sulfonsäure zusetzt, wie sie auch bei einer Veresterung eingesetzt wird.

Ein weiterer Vorteil dieses Verfahrens besteht darin, daß die Itaconsäure und ihre abgewandelte Form, ihr Anhydrid, ebenso wie die im Gemisch vorliegenden Dicarbonsäuren eine zweibasige Säure ist, die sich ohne Rücksicht auf die mit ihr schon abgelaufene Reaktion, z. B. an einer Polyesterharzkondensation beteiligt.

Beispiel 1:

1 kg Rohprodukt aus der Cyclohexanoloxydation mit einem Stickstoffgehalt von 0,007% wird bis zu einer Temperatur von ca. 110°C aufgeschmolzen und dann mit 0,75 g Itaconsäure (stöchiometrische Menge plus 15%) und 0,1 % Benzolsulfonsäure versetzt. Anschließend unterwirft man die Schmelze einer Vakuumdestillation.

Das Destillat zeigt bei Erhitzen auch auf über 210°C über einen längeren Zeitabschnitt (über 6 Std.) keine Dunkelfärbung sondern bleibt etwa bernsteinfarben.

Beispiel 2:

Ein bereits aus dem Cyclohexanoloxydationsprozeß durch z. B. "Ausfrieren" des größten Teils der Adipinsäure gewonnenes Dicarbonsäuregemisch, das bei einer Temperatur bis zu 130°C noch keine Verfärbung zeigt, wird eben bis zu dieser Temperatur aufgeschmolzen. Entsprechend seines Stickstoffgehaltes von 0,14 % wird das Gemisch mit 15 g Itaconsäure (äquivalente Menge plus 15 %) und 0,1 % Benzolsulfonsäure versetzt und nach etwa zehn Minuten mit einem passenden Glycol in der nötigen Menge (z. B. Ethan-1,2-diol) vermischt.

Nach Beendigung des Kondensationsprozesses (nach bekannten Bedingungen) weist das kondensierte Harz ebenso wie das Säuregemisch in Versuch 1 eine Bernsteinfarbe auf.

Beispiel 3 (Vergleichsbeispiel):

Anwendung des gleichen Verfahrens wie in Beispiel 1 jedoch unter Auslassung der Itaconsäure: Bei Erwärmen des Säuregemisches auf über 150°C zeigt sich zusehends eine dunkle, ins rot-schwarze gehende Verfärbung.

Beispiel 4 (Vergleichsbeispiel):

Ansatz wie in Beispiel 2, jedoch ebenso  ohne Itaconsäurezusatz. Das erhaltene Harz zeigt nach der Kondensation eine dunkelrote bis schwarze Färbung.

## Patentansprüche

1. Verfahren zur Verhinderung der Dunkelfärbung von imidhaltigen Produkten und Produktgemischen aufgrund von Wärmeeinwirkungen, d a d u r c h   g e k e n n z e i c h n e t,
   daß man ihrer Schmelze Itaconsäure und/oder Itaconsäureanhydrid zusetzt.

2. Verfahren nach Anspruch 1, dadurch gekennzeichnet, daß
man dem imidhaltigen Produktgemisch, das bei der Oxydation von Cyclohexanol mittels Salpetersäure erhalten wird,
Itaconsäure zusetzt.

3. Verfahren nach Anspruch 1 oder 2, dadurch gekennzeichnet,
daß dem nach Abtrennung des größten Teils der Adipinsäure
erhaltenen Restgemisch Itaconsäure zusetzt.

4. Verfahren nach Anspruch 1 bis 3, dadurch gekennzeichnet, daß
   das Itaconsäure enthaltende Produktgemisch zur Herstellung
   von Polyurethanelastomeren, Preßmassen und Polyestern verwendet wird.

5. Verfahren nach Anspruch 1 bis 4, dadurch gekennzeichnet, daß
   man auf 1 g-Atom Stickstoff in der Schmelze 1 bis 1,5 mol
   Itaconsäure und/oder Itaconsäureanhydrid zusetzt.

6. Verfahren nach Anspruch 1 bis 5, dadurch gekennzeichnet, daß
   man auf 1 g-Atom Stickstoff in der Produktschmelze 1,15 mol
   Itaconsäure und/oder Itaconsäureanhydrd zugibt.

7. Verfahren nach Anspruch 1 bis 6, dadurch gekennzeichnet, daß
   man der Produktschmelze zusätzlich 0,01 % - 5 % bezogen auf
   die Gesamtmenge der Schmelze aromatische Sulfonsäure zusetzt.

- 2 -

8. Verfahren nach Anspruch 1 bis 7, dadurch gekennzeichnet, daß man der Produktschmelze vorzugsweise 0,1 % - 1 % bezogen auf die Gesamtmenge der Schmelze aromatische Sulfonsäure zusetzt.

9. Verfahren nach Anspruch 1 bis 8, dadurch gekennzeichnet, daß die aromatische Sulfonsäure Benzolsulfonsäure oder Toluolsulfonsäure ist.

**Europäisches Patentamt**

**EUROPÄISCHER RECHERCHENBERICHT**

Nummer der Anmeldung

| EINSCHLÄGIGE DOKUMENTE | | | EP 86111094.8 | |
|---|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) | |
| A | DE - B - 1 195 736 (HALCON) <br> * Ansprüche * <br> -- | 1,7-9 | C 07 C 55/02 <br> C 07 C 51/50 <br> C 07 C 55/14 | |
| A | US - A - 4 316 775 (W.D. NASH) <br> * Ansprüche * <br> -- | 1 | | |
| A | US - A - 4 191 616 (B. BAKER) <br> * Ansprüche * <br> -- | 1 | | |
| A | EP - A1 - 0 128 709 (DU PONT CANADA) <br> * Ansprüche * <br> ---- | 1 | | |
| | | | RECHERCHIERTE SACHGEBIETE (Int. Cl.4) | |
| | | | C 07 C 51/00 <br> C 07 C 55/00 | |

Der vorliegende Recherchenbericht wurde für alle Patentansprüche erstellt.

| Recherchenort | Abschlußdatum der Recherche | Prüfer |
|---|---|---|
| WIEN | 11-11-1986 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82